# EUROPÄISCHE PATENTSCHRIFT

(11) **EP 1 033 131 B1**
(45) Veröffentlichungstag und Bekanntmachung des Hinweises auf die Patenterteilung: **19.02.2003**
(21) Anmeldenummer: 98959328.0
(22) Anmeldetag: 18.11.1998
(51) Int. Cl.: A61K 31/375, A61K 31/045, C12G 3/04, C12G 3/06, A61P 25/32

(54) **Zusammensetzung in Form eines Getränks zur Entwicklung einer Aversion gegenüber Alkohol**
Composition in the form of a drink having a delayed alcohol aversion effect
Composition pour boisson ayant un effet d'aversion pour l'alcool

(30) Priorität: 19.11.1997 RU 97118689
(43) Veröffentlichungstag der Anmeldung: 06.09.2000
(73) Patentinhaber: Semenov, Sergei Petrovich, St. Petersburg, 193079 (RU)
(72) Erfinder: Semenov, Sergei Petrovich, St. Petersburg, 193079 (RU)
(74) Vertreter: Springstubbe, Wolf, Dipl.-Ing.
(86) Internationale Anmeldenummer: RU9800388
(87) Internationale Veröffentlichungsnummer: WO99025337

(56) Entgegenhaltungen:
- RU-C1- 2 072 239
- RU-C1- 2 086 237
- US-A- 4 565 689
- US-A- 4 808 574
- US-A- 5 418 255

## Beschreibung

### GEBIET DER TECHNIK

Die Erfindung betrifft die Entwicklung von Zusammensetzungen, die in der Psychopharmakologie und in der Narkologie verwendet werden und therapeutische Eigenschaften besitzen, insbesondere in der Vorbeugung der Ausbildung von Alkoholismus, seiner Prophylaxe und Behandlung.

### BISHERIGER STAND DER TECHNIK

Zur Zeit vermag keines der existierenden Arzneimittel den Alkoholismus zu heilen, weshalb die Suche nach neuen Mitteln zu seiner Prophylaxe und Therapierung aktuell bleibt.

Es sind verschiedene Mittel zur Verminderung einer alkoholischen Intoxikation bekannt, deren Hauptingredienz Bernsteinsäure ist, die ein Naturmetabolikum darstellt, das die Energiespeicherung in den Mitochondrien gewährleistet, was zur Wiederherstellung des energetischen Potentials und zur Verminderung der Alkoholrauschs beiträgt.

Ein Beispiel eines solchen Mittels stellt ein Pfefferminzauszug dar, welcher Bernsteinsäure, Pfefferminzöl und Zucker enthält (RU-Patent Nr. 2012350, A61 K 35/78, 1995).

Es ist eine therapeutische Zusammensetzung zur Beseitigung der Alkohol-Abhängigkeit bekannt (US-Patent Nr. 4 500 515, A61 U 31/ 365, 1985). Die Zusammensetzung umfasst folgende Ingredienzen: Natriumascorbinat 10 bis 15 g, Kalziumgluconat 400 bis 800 mg, Magnesiumchlorid 200 bis 400 mg, Thiaminhydrochlorid 500 bis 1000 mg pro 1000 cm³ einer 0,9%igen Salzlösung. Die Behandlung zur Beseitigung der Alkohol-Abhängigkeit beginnt mit der Einnahme von oralen Dosen von Natrium-, Kalzium- und Magnesiumascorbinat, Vitamin B₁₂; danach wird eine parenterale Aufnahme der genannten therapeutischen Zusammensetzung in einer Menge von 1000 cm³ innerhalb von 2 bis 4 Stunden vorgeschrieben. Eine Entgiftung des Organismus wird innerhalb von 8 bis 20 Stunden erreicht.

Es ist ein Alkoholgetränk bekannt, das prophylaktische therapeutische Eigenschaften und eine antimikrobielle Wirkung aufweist (RU-Patent Nr. 2059703, C12G 3/06, 1996). Das Getränk enthält eine 40%ige Wasser-Alkohol-Flüssigkeit, Vanillin, Zucker, Citronensäure, Hydrophilextrakt aus Walnußblättern, Couleur und Ascorbinsäure in einer Menge von 0,6 bis 1,0 kg pro 1000 dal der Wasser-Alkohol-Flüssigkeit. Das erhaltene Getränk besitzt einen eigenartigen brennenden Geschmack, einen balsamartigen Geruch. Die therapeutischen Eigenschaften des Getränks sind auf die qualitative Zusammensetzung der Ingredienzen, insbesondere auf das Vorhandensein der Ascorbinsäure in der Zusammensetzung zurückzuführen.

Es ist bekannt, dass Ascorbinsäure und deren Derivate den Organismus vor der Bildung von O-Radikalen schützen oder mit O-Radikalen reagieren, bevor diese irreversible Schäden im Organismus hervorrufen, sowie freie Radikale von Peroxiden desaktivieren, die unerwünschte, mit dem Alterungsprozess in Verbindung stehende chemische Reaktionen in lebenden Zellen beschleunigen. Die Zugabe von Ascorbinsäure ermöglicht es, die Ausscheidung von Alkaloiden mit dem Harn zu erhöhen, und unterstützt deshalb die Behandlung von Krebserkrankungen (Melen'teva T.A., Taber A.M.: "Gewinnung von physiologisch wirksamen Stoffen auf der Grundlage der Ascorbinsäure", Moskau, WNIISENTI, 1990, Heft 3).

### OFFENBARUNG DER ERFINDUNG

Die Erfindung ist auf die Schaffung einer Zusammensetzung gerichtet, die im Organismus die negative Wirkung von Ethylalkohol und von toxischen Produkten seiner Oxidation aktiv beeinflusst.

Zweck der Erfindung ist die Schaffung eines Getränks mit guten organoleptischen Eigenschaften, das einen aufgeschobenen Aversionseffekt (Effekt eines Widerwillens gegenüber Alkohol) aufweist, das der Ausbildung des Alkoholismus vorbeugt und zu einer Selbsttherapierung davon beiträgt.

Untersuchungen haben gezeigt, dass die Anwendung des entwikkelten Getränks eine Senkung der Tiefe des durch Ethylalkohol hervorgerufenen narkotischen Schlafs sowie eine Verminderung von Erscheinungen eines Abstinenzsyndroms und der Schwere seines Verlaufs bewirkt.

Eine Prüfung an Personen, die Alkohol missbrauchen, hat gezeigt, dass das Getränk eine spontane aufgeschobene Aversion hervorruft und auf diese Weise zur Selbsttherapierung von Alkoholismus beiträgt.

Es wird eine Zusammensetzung von Ingredienzen vorgeschlagen, enthaltend Ascorbinsäure und/oder deren Derivate, Kochsalz und Wasser-Alkohol-Flüssigkeit in folgendem Verhältnis von Ingredienzen pro 1000 dal des fertigen Produkts:

| | | |
|---|---|---|
| Ascorbinsäure und/oder deren Derivate, | kg | 3 bis 20 |
| Zucker, | kg | 150 bis 250 |
| Kochsalz, | kg | 5 bis 20 |
| 20 - 40%ige Wasser-Alkohol-Flüssigkeit, | 1 | Rest. |

Als Derivate der Ascorbinsäure werden Dehydroascorbinsäure, Ascorbinate von Alkalimetallen oder Erdalkalimetallen, Ethylester der Ascorbinsäure verwendet. Die genannten Ester werden aus einer aus 2,6-Diethylester der Ascorbinsäure, 3,6-Diethylester der Ascorbinsäure, 2,3-Diethylester der Ascorbinsäure, 1-Ethylester der Ascorbinsäure bestehenden Gruppe gewählt.

Ascorbinsäure hat durch ihre Beteiligung an der Regulierung von Oxidations-Reduktions-Prozessen im Kohlenwasserstoffwechsel große Bedeutung für die Lebensvorgänge im Organismus, sie normalisiert die Permeabilität der Kapillaren, ist an der Bildung von Steroidhormonen beteiligt, erhöht die Widerstandsfähigkeit des Organismus gegen Infektionen.

Dehydroascorbinsäure ist ein Derivat der Ascorbinsäure (Lexikon der organischen Verbindungen, Bd. 1, 1949, Moskau, Inostrannaja Literatura, Seite 618). Aus der Literatur ist bekannt, dass Dehydroascorbinsäure und auf deren Grundlage erhaltene Ester eine Aktivität gegen Geschwülste besitzen (Melen'teva T.A., Taber A.M.: "Gewinnung von physiologisch wirksamen Stoffen auf der Grundlage der Ascorbinsäure", Moskau, WNIISENTI, 1990, Heft 3).

Ester der Ascorbinsäure sind die zahlreichsten Derivate der Ascorbinsäure. Ascorbinsäure hat vier Hydroxylgruppen, von denen jede verestert werden kann. Viele Ester der Ascorbinsäure besitzen eine biologische Wirkung, die sich von der biologischen Wirkung der Ascorbinsäure selbst unterscheidet; die Art und das Maß dieser Wirkung hängen vom Aufbau des Esters ab.

Es wurde experimentell festgestellt, dass Ester der Ascorbinsäure besondere Eigenschaften besitzen und zur Korrektur des Befindens bei Alkoholtrunkenheit und eines Alkohol-Entzugssyndroms sowie zur Verminderung von Dauer und Stärke der Anregungswirkung des Alkohols und zur Verlangsamung des Eintritts der Phase der Alkoholanregung verwendet werden können (RU-Patent Nr. 2112510, A61K 31/375, 1998). Vor dem Hintergrund einer Zwangsalkoholisierung hat die Verabreichung von Ethylestern der Ascorbinsäure in einer Dosis von 0,4 g/kg täglich für 7 Tage zu einer Senkung von Erscheinungen eines Abstinenzsyndroms bei Ratten geführt.

Experimentelle Daten über den Charakter der Verhaltensreaktionen der untersuchten Tiere zeigen, dass bei eingeschwungenem Pegel des Alkoholverbrauchs die Merkmale der lokomotorischen Aktivität eine Tendenz zur Erhöhung haben. Unter diesen Umständen senkt die Verwendung der Zusammensetzung diese Merkmale.

2,6-Diethylester der Ascorbinsäure vermindert die Verlaufsschwere des Alkoholentzugssyndroms, vermindert die Stärke der alkoholischen Anregung mit dem Quotienten 4 und die Dauer um 25%, vermindert die Dauer des Auftretens des narkotischen Effekts des Alkohols, verlangsamt das Eintreten der Anregungsphase.
2,3-Diethylester der Ascorbinsäure vermindert die Verlaufsschwere des Alkoholentzugssyndroms, beeinflusst den Anregungseffekt der Alkoholwirkung.
3,6-Diethylester der Ascorbinsäure vermindert die Dauer der narkotischen Wirkung des Alkohols.
1-Ethylester der Ascorbinsäure vermindert die Aktivität der Cholinesterase mit mehr als dem Quotienten 2. Es hat sich seine Fähigkeit gezeigt, nicht nur die alkoholische Anregung zu vermindern, sondern auch den Zeitraum der Anregung mit dem Quotienten 2 zu verkürzen.

Alle Ethylester der Ascorbinsäure sind wenig toxische Verbindungen. Bei einmaliger innerer Verabreichung betrug die Größe LD 50 nicht mehr als 5 g/kg, weshalb diese Stoffe der Klasse IV der wenig gefährlichen Verbindungen zugeordnet wurden.

Ascorbinsäure und deren Derivate schützen den Organismus gegen die Bildung von O-Radikalen oder reagieren mit O-Radikalen, bevor diese irreparable Schäden im Organismus hervorrufen, desaktivieren freie Radikale von Peroxiden, die unerwünschte chemische Reaktionen in lebenden Zellen beschleunigen.

Für prophylaktische und therapeutische Zwecke ist die Verabreichung einer Dosis von 0,05 bis 0,1 g Ascorbinsäure 3 bis 5 mal täglich üblich. Statt Ascorbinsäure werden 1 bis 3 ml einer 5%igen Natriumsascorbinatlösung parenteral verabreicht.

In dem entwickelten Getränk sind Ascorbinsäure und/oder deren Derivate in einer erhöhten Menge vorhanden, was zusammen mit den anderen Ingredienzen die Schaffung eines Getränks mit guten organoleptischen Eigenschaften sicherstellt, das einen aufgeschobenen Aversionseffekt besitzt, der Entwicklung des Alkoholismus vorbeugt und zur Selbsttherapierung davon beiträgt.

In dem entwickelten Getränk spielen Ascorbinsäure und deren Derivate eine therapeutisch-prophylaktische Rolle und die Rolle von Konservierungsstoffen, und Zucker und Salz verstärken diese ihre Funktion.

Natriumionen, die sich im Getränk bei der Dissoziation des Kochsalzes bilden, stellen seine Wirkung auf das zentrale Nervensystem sicher.

Eine Senkung des Anteils der Ascorbinsäure und/oder ihrer Derivate unter 3 kg pro 1000 dal des fertigen Produkts führt zu einem Verschwinden des aufgeschobenen Aversionseffekts. Die Anwendung der genannten Ingredienzen in einer Menge von über 20 kg bringt das Risiko einer Hypervitaminose mit Vitamin C mit sich.

Bei der Herstellung des Getränks wird eine Sortierung aus rektifiziertem Ethylalkohol der Sorte "Extra" oder "Luxus" und Wasser verwendet. In diese Sortierung werden zuvor in Wasser gelöste Zucker und Salz zugegeben, wobei die Wassermenge im Hinblick auf die Zubereitung einer Wasser-Spiritus-Flüssigkeit vorgegebener Stärke genommen wird. Der erhaltene Verschnitt wird in Kohlefiltern gefiltert, und dann wird in diesen eine vorgegebene Menge Ascorbinsäure oder ihrer Derivate zugegeben. Danach wird der Verschnitt für 2 bis 4 Stunden stehengelassen und nochmals gefiltert.

Untersuchungen der Toxizität des Getränks, seines Einflusses auf die narkotische Wirkung des Ethylalkohols und auf den Verlauf des Alkoholentzugssyndroms wurden an weißen Ratten durchgeführt. Im scharfen Experiment wurde den Ratten die entwickelte Rezeptur einmalig in einer Dosis von 20 ml/kg intragastral verabreicht, was bezogen auf den Ethylalkohol 10 g/kg ausmacht. Bei den Versuchstieren hat sich schnell ein Zustand einer schweren Alkoholtrunkenheit ausgebildet. Letale Ausgänge wurden innerhalb von zwei Wochen nach der einmaligen Verabreichung von Alkohol nicht festgestellt.

Die Toxizität der Zusammensetzung wurde bei deren dauerhafter Verabreichung bestimmt. Zu diesem Zweck wurde die Zusammensetzung während eines Monats 5 mal in der Woche täglich in Form einer wässrigen Lösung in einer Verdünnung von 1:1 verabreicht, was 0,7 ml Ethylalkohol pro kg entspricht. Im Laufe der Untersuchungen wurden der allgemeine Zustand der Tiere, die spontane Bewegungsaktivität, der Charakter von Verhaltensreaktionen, das Verhalten gegenüber Wasser und Nahrung registriert und makroskopische Auswertungen des Zustandes der inneren Organe durchgeführt.

Die Untersuchungen haben gezeigt, dass bei dem Getränk toxische Anzeichen einer schädigenden Wirkung auf die inneren Organe (Herz, Nieren, Leber) fehlen. Die dauerhafte Einwirkung des Getränks hat die Werte der Erythrozyten, Monozyten und des Hämoglobins im Blut der Ratten nicht verändert. Die klinische Urinprobe hat gezeigt, dass im Urin keine pathologischen Komponenten (Zucker, Keton- und Urobilinkörper) vorhanden sind.

Zur Auswertung des Einflusses des Alkoholgetränks auf den Verlauf des Abstinenzsyndroms (Syndrom des Alkoholentzugs) wurde den Ratten innerhalb von 7 Tagen innerlich eine Rezeptur in einer Menge verabreicht, die einer Ethylalkohol-Dosis von 4 g/kg entspricht. Der Verlauf des Syndroms wurde nach der D. Goldstein-Methodik (Goldstein D.B., NATURE, 245, 154 - 156, 1973) charakterisiert. Als Grundlage der Testierung diente der Zustand der Verhaltensreaktionen der Ratten unter der Einwirkung von Licht als Erregungsfaktor.

Die durchgeführten Untersuchungen haben gezeigt, dass, wenn die Schwere des Verlaufs des Abstinenzsyndroms bei einer Alkoholisierung unterzogenen Ratten mehr als 23 Stufen betragen hat, sie bei Verwendung des entwickelten Getränks 2 Stufen nicht überschritten hat.

Es wurde eine Auswertung des Verlaufs der Ethanolnarkose durchgeführt. Ethylalkohol und die untersuchte Rezeptur wurden in einer Äquivalenzmenge entsprechend 5 g/kg verabreicht. Der narkotische Effekt wurde hinsichtlich der Latenzperiode des Eintretens des Schlafs und dessen Dauer und Tiefe ausgewertet. Die Verwendung der entwickelten Rezeptur hat das Eintreten des narkotischen Schlafs verzögert und seine Dauer um die Hälfte verkürzt. Vor dem Hintergrund der Verabreichung des Getränks war die Tiefe des narkotischen Schlafs nicht so stark.

### VARIANTEN DER REALISIERUNG DER ERFINDUNG

### BEISPIEL 1.

Zur Zubereitung des Getränks werden Ethylalkohol der Rektifikationssorte "Luxus", Trinkwasser eines Härtegrades von bis zu 0,1 Mol/m³, feiner Raffinade-Zucker, Kochsalz, Lebensmittel-Ascorbinsäure verwendet.
Zur Zubereitung von 1000 dal des fertigen Produkts werden die genannten Ingredienzen in folgenden Mengen verwendet:

| | |
|---|---|
| Ascorbinsäure | 10 kg |
| Zucker | 150 kg |
| Kochsalz | 10 kg |
| 40%ige Wasser-Alkohol-Flüssigkeit, | Rest 1. |

Bei der Zubereitung des Getränks wird eine Sortierung aus rektifiziertem Ethylalkohol und Wasser verwendet. Dieser Sortierung werden zuvor in Wasser gelöste 150 kg Zucker und 10 kg Salz zugefügt, wobei die Wassermenge im Hinblick auf die Zubereitung einer 40%igen Wasser-Spiritus-Flüssigkeit genommen wird. Der erhaltene Verschnitt wird in Kohlefiltern gefiltert, und dann werden in diesen 10 kg Ascorbinsäure zugegeben. Danach wird der Verschnitt für 2 bis 4 Stunden stehengelassen und nochmals gefiltert.

### BEISPIEL 2.

Zur Zubereitung von 1000 dal des fertigen Produkts werden rektifizierter Alkohol, Sorte "Extra", und folgende Ingredienzen verwendet:

| | |
|---|---|
| Ascorbinsäure | 15 kg |
| Zucker | 250 kg |
| Kochsalz | 25 kg |
| 40%ige Wasser-Alkohol-Flüssigkeit, | Rest 1. |

### BEISPIEL 3.

Zur Zubereitung von 1000 dal des fertigen Produkts werden rektifizierter Alkohol, Sorte "Extra", und folgende Ingredienzen verwendet:

| | |
|---|---|
| Dehydroascorbinsäure | 20 kg |
| Zucker | 250 kg |
| Kochsalz | 20 kg |
| 40%ige Wasser-Alkohol-Flüssigkeit, | Rest 1. |

### BEISPIEL 4.

Zur Zubereitung von 1000 dal des fertigen Produkts werden rektifizierter Alkohol, Sorte "Luxus", und folgende Ingredienzen verwendet:

| | |
|---|---|
| Ascorbinsäure | 5 kg |
| Natriumascorbinat | 10 kg |
| Zucker | 250 kg |
| Kochsalz | 5 kg |
| 20%ige Wasser-Alkohol-Flüssigkeit, | Rest 1. |

### BEISPIEL 5.

Zur Zubereitung von 1000 dal des fertigen Produkts werden rektifizierter Alkohol, Sorte "Extra", und folgende Ingredienzen verwendet:

| | |
|---|---|
| 2,6-Diethylester der Ascorbinsäure | 15 kg |
| Zucker | 250 kg |
| Kochsalz | 5 kg |
| 20%ige Wasser-Alkohol-Flüssigkeit, | Rest 1. |

### BEISPIEL 6.

Zur Zubereitung von 1000 dal des fertigen Produkts werden rektifizierter Alkohol, Sorte "Extra", und folgende Ingredienzen verwendet:

| | |
|---|---|
| Ascorbinsäure | 5 kg |
| 2,3-Diethylester der Ascorbinsäure | 10 kg |
| Zucker | 250 kg |
| Kochsalz | 5 kg |
| 20%ige Wasser-Alkohol-Flüssigkeit, | Rest 1. |

Die erhaltenen Getränke haben folgende organoleptische Merkmale: Äußeres: durchsichtige Flüssigkeit ohne Fremdeinschlüsse; Farbe: farblose Flüssigkeit; Geschmack: schwach süßsäuerlich ohne Fremdgeschmack und Aroma.

### GEWERBLICHE VERWERTBARKEIT

Das entwickelte Getränk, das nach dem Beispiel 2 hergestellt wurde, wurde einer alkoholische Getränke missbrauchenden männlichen Person D., 45 Jahre, angeboten. D. hat es statt der gewöhnlichen alkoholischen Getränke bis zu einem dem zweiten Trunkenheitsgrad entsprechenden Zustand zu sich genommen. Nach Zusichnahme des genannten Getränks während zweier Monate gemäß seinen Alkoholisierungsgewohnheiten hat sich bei D. eine spontane Aversion (Widerwille) gegen den Zustand der alkoholischen Trunkenheit entwickelt, ohne allgemeine Erschwerung der klinischen Erscheinungen des Alkoholismus.

Bei Konsum des entwickelten Getränks in nicht zu Trunkenheit zweiten oder dritten Grades führenden Dosen zeichnet sich das Befinden der Person bei Zusichnahme des Getränks durch eine stärker ausgeprägte Euphorie und eine Verminderung der Erscheinungen des Abstinenzsyndroms aus.

Somit kann auf der Grundlage der toxikologischen Prüfungen und der experimentellen Erprobung das entwickelte, gute organoleptische Eigenschaften aufweisende Getränk zur Prophylaxe und Selbsttherapierung des Alkoholismus empfohlen werden.

## Patentansprüche

1. Zusammensetzung in Form eines Getränks zur Entwicklung einer Aversion gegenüber Alkohol, enthaltend 20 - 40%ige Wasser-Alkohol-Flüssigkeit aus Ethylalkohol und Wasser, Ascorbinsäure und/oder deren Derivate, Zucker und Kochsalz bei folgendem Verhältnis der Ingredienzen pro 1000 dal des fertigen Produkts:
| | | |
|---|---|---|
| Ascorbinsäure und/oder deren Derivate, | kg | 3 bis 20 |
| Zucker, | kg | 150 bis 250 |
| Kochsalz, | kg | 5 bis 20 |
| 20 - 40%ige Wasser-Alkohol-Flüssigkeit, | 1 | Rest, |
wobei die Zusammensetzung als Derivate der Ascorbinsäure
- Dehydroascorbinsäure und/oder
- Ascorbinate von Alkalimetallen oder Erdalkalimetallen und/oder
- Ethylester der Ascorbinsäure enthält.

2. Zusammensetzung nach Anspruch 1,
**dadurch gekennzeichnet,**
**daß** die Ethylester gewählt sind von 2,6-Diethylester der Ascorbinsäure, 3,6-Diethylester der Ascorbinsäure, 2,3-Diethylester der Ascorbinsäure, 1-Ethylester der Ascorbinsäure.

## Claims

1. Composition in the form of a beverage for developing an aversion to alcohol, containing 20-40% strength hydroalcoholic fluid composed of ethyl alcohol and water, ascorbic acid and/or derivatives thereof, sugar and sodium chloride in the following ratio of the ingredients per 1 000 dal of the finished product:
| | | |
|---|---|---|
| Ascorbic acid and/or derivatives thereof, | kg | 3 to 20 |
| Sugar, | kg | 150 to 250 |
| Sodium chloride, | kg | 5 to 20 |
| 20-40% strength hydroalcoholic fluid, | l1 | remainder, |
where the composition contains as derivatives of ascorbic acid
- dehydroascorbic acid and/or
- ascorbinates of alkali metals or alkaline earth metals and/or
- ethyl esters of ascorbic acid.

2. Composition according to Claim 1, **characterized in that** the ethyl esters are selected from 2,6-diethyl ester of ascorbic acid, 3,6-diethyl ester of ascorbic acid, 2,3-diethyl ester of ascorbic acid, 1-ethyl ester of ascorbic acid.

## Revendications

1. Composition, sous forme d'une boisson pour le développement d'une aversion vis-à-vis de l'alcool, contenant un liquide hydro-alcoolique à 20-40 %, constitué d'alcool éthylique et d'eau, de l'acide ascorbique et/ou ses dérivés, du sucre et du chlorure de sodium, les ingrédients étant présents selon les proportions suivantes, pour 1000 dal du produit fini :
| | |
|---|---|
| acide ascorbique et/ou ses dérivés, kg | 3 à 20 |
| sucre, kg | 150 à 250 |
| chlorure de sodium, kg | 5 à 20 |
| liquide hydro-alcoolique à 20-40 %, 1 | le reste |
la composition contenant, en tant que dérivés de l'acide ascorbique
- de l'acide déshydroascorbique et/ou
- des ascorbates de métaux alcalins ou de métaux alcalino-terreux, et/ou
- des esters éthyliques de l'acide ascorbique.

2. Composition selon la revendication 1, **caractérisée en ce que** les esters éthyliques sont choisis parmi l'ester 2,6-diéthylique de l'acide ascorbique, l'ester 3,6-diéthylique de l'acide ascorbique, l'ester 2,3-diéthylique de l'acide ascorbique, l'ester 1-éthylique de l'acide ascorbique.
